# EUROPÄISCHE PATENTSCHRIFT

(11) **EP 0 492 328 B1**
(45) Veröffentlichungstag und Bekanntmachung des Hinweises auf die Patenterteilung: **07.06.1995**
(21) Anmeldenummer: 91121481.5
(22) Anmeldetag: 14.12.1991
(51) Int. Cl.: A61F 13/06

(54) **Kniebandage**
Knee bandage
Genouillère

(30) Priorität: 28.12.1990 DE 9017540 U
(43) Veröffentlichungstag der Anmeldung: 01.07.1992
(73) Patentinhaber: FERD. HAUBER GmbH & CO. KG, D-72604 Nürtingen (DE)
(72) Erfinder: Berkowitsch, Ewald, W-7312 Kirchheim/Teck (DE)
(74) Vertreter: Becker, Maria, Dipl.-Phys.

(56) Entgegenhaltungen:
- EP-A- 0 010 389
- DE-U- 8 115 670
- FR-A- 2 636 228
- US-A- 3 945 046
- US-A- 4 287 884

## Beschreibung

Die Erfindung betrifft eine Kniebandage mit einer schlauchförmigen Hülle aus elastisch dehnbarem Material, die eine den Patellabereich bogenförmig umgebende, in einer Tasche der Bandage angeordnete Pelotte aufweist, die sich medial öffnet und an deren freien Enden jeweils ein elastisches Spannband befestigt ist, wobei die Spannbänder zum Spannen der Kniebandage jeweils aus einem Schlitz der Tasche herausgeführt und bandagenaussenseitig festlegbar sind.

Eine derartige Kniebandage ist aus DE-U- 81 15 670 bekannt. Bei dieser Bandage ist die Patella-Pelotte C-förmig ausgebildet, wobei die offene Seite medial (beineinwärtig) gerichtet ist und dabei den oberen und unteren Bereich der Kniescheibe umgibt. Von den freien Pelottenenden dieser Bandage gehen Spannbänder aus, die in einem Winkel zusammengeführt und von der Vereinigungsstelle als ein einziges Band um den Bereich der Kniekehle herumgeführt und schliesslich seitlich mittels einer Schnelle festgelegt sind. Es besteht bei einer derartigen Bandführung beim längeren Tragen die Gefahr des Abschnürens im Bereich der Kniekehle und dadurch ein unangenehmes Druckgefühl.

Der Erfindung liegt die Aufgabe zugrunde, diese Nachteile bei einer Kniebandage der bekannten Art zu verbessern und die Spannbänder so anzuordnen, dass sie nicht nur die Bandage um das Knie spannen, sondern auch gleichzeitig eine verbesserte Stützfunktion ausüben.

Diese Aufgabe wird gemäss der Erfindung dadurch gelöst, dass in den Endbereichen der Kniebandage jeweils ein erster, einem der Pelottenenden gegenüberliegend vorgesehener Verschlussteil angeordnet ist und die Spannbänder am freien Ende einen zweiten Verschlussteil tragen, und dass bei angelegter Bandage der zweite Verschlussteil des unteren Spannbandes am oben liegenden ersten Verschlussteil, und das zweite Verschlussteil des oberen Spannbandes am unten liegenden ersten Verschlussteil festgelegt ist. Bei dieser Anordnung werden die Spannbänder jeweils ausgehend von den freien Enden der bogenförmigen Pelotte kreuzweise um die Kniekehle geführt und dann am unteren bzw. oberen Bereich der Bandage festgelegt. Es ergibt sich kein unangenehmes Spanngefühl, sondern gleichzeitig eine Kreuzbandstabilisierung. Auch gestattet diese Bandführung eine bessere Zugregulierung.

Die Medialisierung der Kniescheibe wird gefördert, wenn gemäss einer bevorzugten Ausführungsform der freie Schenkel der bogenförmigen Patella-Pelotte im unteren Bereich länger ausgebildet ist als der obere Schenkel.

Ein Ausführungsbeispiel des Gegenstandes der Erfindung ist in der Zeichnung dargestellt. Hierin zeigen:
- Fig. 1: eine Vorderansicht der Kniebandage,
- Fig. 2: eine Rückansicht hierzu.

Die insgesamt mit 10 bezeichnete Kniebandage besteht aus einer schlauchförmigen Hülle 12 aus einem elastischen Bandagenstoff. Zur Stabilisierung der Bandage können in bekanntor Weise an den Längsseiten geführte Verstärkungen oder Stützen aus federndem Material 14, 16 eingenäht sein.

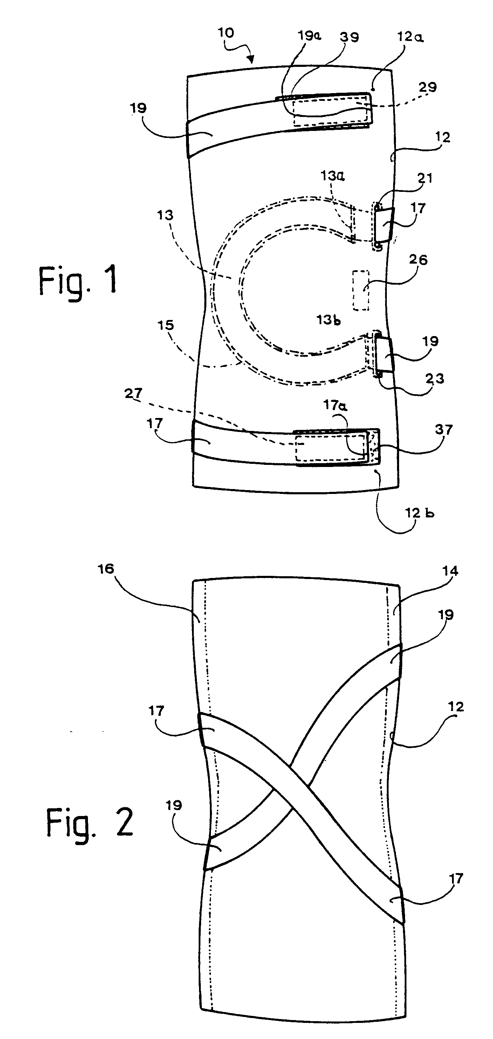

Wie aus Fig. 1 ersichtlich, weist die Bandage 10 im mittleren Bereich ihrer Vorderseite eine im angelegten Zustand die Patella umgebende C-förmige Pelotte 13 auf, die vorzugsweise aus Silikon besteht. Sie ist in einer Tasche 15 der Bandage 12 eingenäht derart, dass sie sich medial öffnet.

An den freien Enden 13a, 13b der Pelotte sind Spannbänder 17, 19 befestigt, die durch Schlitze 21, 23 der Tasche 15 herausgeführt und, wie Fig. 2 zeigt, kreuzweise im angelegten Zustand über die Kniekehle des Benutzers bandagenrückseitig herumgeführt sind. Die freien Enden 17a und 17b der Spannbänder 17, 19 sind mit Verschlussteilen 27, 29 ausgerüstet, die in den Endbereichen 12a, 12b der Bandage mit Gegenverschlussteilen 37, 39 im angelegten Zustand zusammenwirken und die Bänder 17, 19 festlegen. Die Verschlüsse sind vorzugsweise Klettverschlüsse. Die Spannbänder 17, 19 medialisieren die Pelotte und üben dadurch die gewünschte therapeutische Wirkung aus.

Diese wird noch dadurch unterstützt, dass der untere freie Schenkel 13b der bogenförmigen Patella-Pelotte 13 im unteren Bereich länger ausgebildet ist als der obere Schenkel 13a, und so über das Ligamentumpatellae verlängert ist.

Zur verbesserten therapeutischen Wirkung kann noch ferner vorgesehen sein, dass im Bereich der Öffnung der C-förmigen Pelotte 13 in Weiterführung des C-Bogens ein weiteres Pelottensegment 26 in einer Tasche eingelegt ist.

Die beschriebene Pelotte eignet sich insbesondere für eine passive und aktiv-dynamische Kniescheibenmedialisierung. Sie zeichnet sich durch angenehmes Tragen aus, da sie praktisch nicht einschnürt.

## Patentansprüche

1. Kniebandage (10) mit einer schlauchförmigen Hülle (12) aus elastisch dehnbarem Material, die eine den Patellabereich bogenförmig umgebende, in einer Tasche (15) der Bandage (10) angeordnete Pelotte (13) aufweist, die sich medial öffnet und an deren freien Enden (13a, 13b) jeweils ein elastisches Spannband (17, 19) befestigt ist, wobei die Spannbänder (17, 19) zum Spannen der Kniebandage (10) jeweils aus einem Schlitz der Tasche (15) herausgeführt und bandagenaussenseitig festlegbar sind,
**dadurch gekennzeichnet,**
dass in den Endbereichen (12a, 12b) der Kniebandage (10) jeweils ein erster, einem der Pelottenenden (13a, 13b) gegenüberliegend vorgesehener Verschlussteil (37, 39) angeordnet ist und die Spannbänder (17, 19) am freien Ende (17a, 19a) einen zweiten Verschlussteil (27, 29) tragen, und dass bei angelegter Bandage der zweite Verschlussteil (29) des unteren Spannbandes (19) am oben liegenden ersten Verschlussteil (39), und das zweite Verschlussteil (27) des oberen Spannbandes (17) am unten liegenden ersten Verschlussteil (37) festgelegt ist.

2. Kniebandage nach Anspruch 1, dadurch gekennzeichnet, dass die Verschlussteile (27, 29; 37, 39) Klettverschlüsse bilden.

3. Kniebandage nach Anspruch 1, dadurch gekennzeichnet, dass der freie Schenkel (13b) der bogenförmigen Patella-Pelotte (13) im unteren Bereich länger ausgebildet ist als der obere Schenkel (13a).

4. Kniebandage nach Anspruch 1, dadurch gekennzeichnet, dass im Bereich der Öffnung der C-förmigen Pelotte (13) in Weiterführung des C-Bogens ein weiteres Pelottensegment (26) in einer Tasche eingelegt ist.

## Claims

1. Knee-bandage (10) having a hose-like sheath (12) made from an elastically extensible material, that encloses a truss pad (13) located in a pocket (15) of the bandage (10) and encircling the patella area along an arcuate path, opening medially and having fixed to each of its free ends (13a, 13b) an elastic tensioning strap (17, 19), the tensioning straps (17, 19) being arranged in such a way that for tightening the knee-bandage (10) each of them can be withdrawn from the pocket (15) through a slot and fixed on the outside of the bandage,
**characterized in that**
there are provided, in the end portions (12a, 12b) of the knee-bandage (10), first closure members (37, 39) each arranged opposite one end of the truss pad (13a, 13b), respectively, that each of the tensioning straps (17, 19) carries on its free end (17a, 19a) a second closure member (27, 29), and that after fitting of the bandage, the second closure member (29) of the lower tensioning strap (19) is fixed on the upper one (39) of said first closure members, while the second closure member (27) of the upper tensining strap (17) is fixed on the lower one (37) of said first closure members.

2. Knee-bandage according to claim 1, characterized in that the closure members (27, 29; 37, 39) take the form of velcro fastenings.

3. Knee bandage according to claim 1, characterized in that the free leg (13b) of the arc-shaped patella truss pad (13) in the lower area is longer than the upper leg (13a).

4. Knee-bandage according to claim 1, characterized in that another truss pad segment (26) is inserted in a pocket in the area of the opening of the C-shaped truss pad (13) so as to continue the C shape of that pad.

## Revendications

1. Bandage de genou (10) avec une gaine flexible (12) en un matériau extensible élastiquement présentant une pelote (13), logée dans une poche (15) du bandage (10), entourant en forme d'arche la zone de la rotule et s'ouvrant médialement et aux extrémités (13a, 13b) de laquelle est fixée respectivement une bande de serrage élastique (17, 19), les bandes de serrage (17, 19) pour le serrage du bandage de genou (10) sortant d'une fente de la poche (15) et pouvant être fixées sur la face extérieure du bandage,
**caractérisé en ce que**
sur les zones d'extrémité (12a, 12b) du bandage de genou (10) est disposé respectivement un élément de fermeture (37, 39), opposé aux extrémités de la pelote (13a, 13b), et que les bandes de serrage (17, 19) portent sur leur extrémité libre (17a, 19a) un deuxième élément de fermeture (27, 29), et que, une fois le bandage appliqué, le deuxième élément de fermeture (29) de la bande de serrage inférieure (19) est fixé sur le premier élément de fermeture (39) du haut et le deuxième élément de fermeture (27) de la bande de serrage supérieure (17) est fixé sur le premier élément de fermeture (37) du bas.

2. Bandage de genou selon la revendication 1, caractérisé en ce que les éléments de fermeture (27, 29; 37, 39) sont conçus comme fermetures Velcro.

3. Bandage de genou selon la revendication 1, caractérisé en ce que l'aisselle libre (13b) de la pelote de rotule en forme d'arche (13) est plus longue dans la zone inférieure que l'aisselle supérieure (13a).

4. Bandage de genou selon la revendication 1, caractérisé en ce que dans la zone de l'ouverture de la pelote en forme de C (13) un autre segment de pelote (26) est, comme prolongement de l'arche en C, logé dans une poche.
